# FASCICULE DE BREVET EUROPEEN

(11) **EP 1 409 002 B1**
(45) Date de publication et mention de la délivrance du brevet: **15.08.2007**
(21) Numéro de dépôt: 02791495.1
(22) Date de dépôt: 10.07.2002
(51) Int. Cl.: A61K 9/16, A61K 36/84, A61K 36/87, A61K 36/288

(54) **SPHEROIDES A BASE D'ADSORBATS D'EXTRAITS VEGETAUX ET PROCEDE DE PREPARATION**
SPHÄROIDE AUS ADSORBATEN VON PFLANZENEXTRAKTEN UND HERSTELLUNGSVERFAHREN
SPHEROIDS BASED ON PLANT EXTRACT ADSORBATES AND METHOD FOR PREPARING SAME

(30) Priorité: 25.07.2001 FR 0109912
(43) Date de publication de la demande: 21.04.2004
(73) Titulaire: Societe Civile d'Inventeurs Apis Spheromont, 67100 Strasbourg (FR)
(72) Inventeur: JACOB, Maurice, F-34000 Montpellier (FR); BATAILLE, Bernard, F-34980 Saint Gely du Fesc (FR); JACOB, Olivier, F-34090 Montpellier (FR); IDERNE, Michel, F-67100 Strasbourg (FR)
(74) Mandataire: Metz, Paul
(86) Numéro de dépôt international: PCT/FR2002/002425
(87) Numéro de publication internationale: WO 2003/011312

(56) Documents cités:
- EP-A- 0 062 893
- EP-A- 0 539 304
- EP-A- 0 689 833
- DATABASE WPI Section Ch, Week 198604 Derwent Publications Ltd., London, GB; Class D13, AN 1986-023649 XP002194486 & JP 60 244271 A (AJINOMOTO KK), 4 décembre 1985 (1985-12-04)

## Description

La présente invention concerne la formulation de sphéroïdes à base d'éther de cellulose hydroxypropylé faiblement substitué et pouvant être fortement chargés en substances actives et permettant leur libération rapide dans un milieu donné.

Elle concerne également le procédé de préparation de ces sphéroïdes à base d'éther de cellulose hydroxypropylé faiblement substitué qui permet d'apporter une solution active, notamment une solution d'origine végétale, en quantité très concentrée sur un sphéroïde.

L'invention concerne également les compositions végétales, minérales, vitaminiques ou à base de composants actifs organiques, sous la forme de sphéroïdes de ce type.

Enfin, elle concerne les applications qui en découlent pour l'administration de ces sphéroïdes par voie interne ou externe et notamment dans les domaines de la santé, de l'hygiène, de la diététique, de la cosmétologie, de la nutrition, de l'agriculture, pour l'homme ou l'animal.

L'un des buts de l'invention est de fournir des sphéroïdes fortement titrés en principes actifs.

Un autre but de la présente invention est de fournir des sphéroïdes à libération rapide des substances actives.

Il est bien connu de préparer des solutions actives sous forme liquide et notamment des solutions à base de produits végétaux obtenus par extraction, macération, infusion, décoction, digestion ou lixiviation.

Cependant, l'utilisation directe de ces extraits liquides présente de nombreux inconvénients notamment quant à leur instabilité physique et chimique au cours de la conservation, quant à leur faible teneur en constituants végétaux caractéristiques et quant à la présence fréquente d'éthanol en quantité plus ou moins importante ce qui n'est généralement pas souhaitable en particulier pour l'administration par voie orale de produits médicamenteux.

En outre, la forme liquide est peu pratique pour l'utilisateur. La prise et le dosage du produit sont compliqués, son transport difficile et il peut accidentellement être renversé.

Pour toutes ces raisons, une forme solide est fortement souhaitable.

Actuellement, pour transformer ces extraits liquides en extraits secs présentant moins d'inconvénients, on utilise des procédés nécessitant d'importants apports calorifiques comme par exemple la nébulisation, le séchage sur cylindres rotatifs ou l'évaporation sous pression réduite.

Si ces procédés permettent d'obtenir des composés secs sous forme de poudres plus faciles à utiliser et notamment administrables par voie orale, ils font appel à des températures élevées qui peuvent altérer les principes actifs fragiles comme par exemple les constituants caractéristiques de végétaux.

De plus, ces extraits secs sont très souvent hygroscopiques, ce qui conduit par reprise d'humidité à du mottage, à des risques de modification de la stabilité physique et chimique, à des difficultés de reproductibilité et de manipulation.

On connaît également le procédé décrit dans la demande de brevet FR 2.721.512 qui consiste à absorber et à adsorber une solution d'origine végétale sur une substance généralement en poudre de type polymères naturels ou synthétiques, à extruder puis à sphéroniser la masse humidifiée afin de former des sphéroïdes.

Par ce procédé, on obtient avantageusement sans dégradation thermique des formulations simplifiées permettant l'administration sous forme sèche de préparations liquides d'origine végétale et assurant la stabilité physique et chimique dans le temps des principes actifs utilisés.

Les polymères décrits dans cette antériorité comme présentant des propriétés absorbantes et adsorbantes convenables sont les celluloses microcristallines, les celluloses microfines, les amidons, les amidons modifiés et les polysaccharides, la cellulose microcristalline étant préférée. Ces excipients technologiques présentent des caractéristiques plastiques satisfaisantes et des propriétés d'absorption et d'adsorption convenables.

Cependant, il est particulièrement avantageux d'améliorer ces propriétés afin de réaliser des sphéroïdes plus fortement concentrés en principes actifs.

En effet, il est alors possible de charger davantage de principe actif dans un système distributeur plus petit. On peut ainsi, par exemple dans le cadre d'une administration par voie orale, diminuer le nombre de prises dans la journée ou le volume de composition à avaler par prise.

Ces objectifs ont été atteints grâce à la formulation et au procédé selon l'invention.

Pour cela et selon une caractéristique essentielle de l'invention, on utilise un excipient technique spécifique qui de façon surprenante s'est révélé être à la fois particulièrement avantageux comme substrat d'absorption et d'adsorption pour les principes actifs et compatible avec le procédé d'extrusion sphéronisation.

Les sphéroïdes selon l'invention sont formulés à partir d'au moins une substance active absorbée et/ou adsorbée sur un mélange technologique homogène sec de polymère cellulosique hydroxypropylé faiblement substitué et sont obtenus par un procédé d'extrusion sphéronisation.

Le procédé de fabrication de sphéroïdes fortement titrés en principes actifs selon l'invention comprend les étapes suivantes :
- fournir une solution ou une poudre active très concentrée en principes actifs ;
- fournir un mélange technologique homogène sec de polymère cellulosique hydroxypropylé faiblement substitué à propriétés absorbantes et adsorbantes très élevées ;
- effectuer l'humidification du mélange sec par la solution active ou par un autre liquide aqueux ou non aqueux ;
- extruder ;
- former des sphéroïdes par sphéronisation du produit extrudé ;
- sécher ;
- calibrer les sphéroïdes secs pour former des systèmes multiparticulaires sphériques de granulométrie maîtrisée.

Le procédé selon l'invention peut comprendre une étape supplémentaire qui consiste à :
- pelliculer ces systèmes multiparticulaires afin par exemple de protéger les constituants ou de modifier leur libération dans l'organisme.

Le procédé selon l'invention permet d'apporter toute composition végétale active sous forme liquide, notamment un extrait liquide, une solution, une suspension, ou sous forme solide par exemple une poudre simple ou complexe, un extrait sec ou autre, sur un sphéroïde de granulométrie maîtrisée.

Il s'adresse de préférence à un extrait alcoolique ou hydro-alcoolique d'une ou plusieurs matières premières végétales. Cependant, il peut également convenir à toute autre solution ou poudre active contenant un ou plusieurs principes actifs, par exemple à base de vitamines, de minéraux et/ou de composants organiques.

L'une des caractéristiques de l'invention est d'utiliser pour le mélange technologique sec les propriétés physico-chimiques particulières d'un éther de cellulose hydroxypropylé faiblement substitué au niveau des groupes du noyau β-O-glucopyranosile. Ce composé est de préférence caractérisé par un taux de substitution (groupements hydroxypropyls) de l'ordre de 10% par exemple voisin de 11% et de manière préférentielle par un pourcentage de fraction soluble dans l'eau de l'ordre de 5% par exemple voisin de 4,29%.

Ce composé présente des propriétés plastiques, absorbantes et adsorbantes des liquides, compatibles avec le procédé d'extrusion et de sphéronisation. De plus, il permet l'élaboration de sphéroïdes beaucoup plus concentrés en composants apportés, comparativement à ceux préparés par le même procédé à partir d'excipients technologiques plus conventionnels, comme notamment la cellulose microcristalline.

A titre d'exemple, on peut citer les réalisations suivantes qui justifient cette affirmation, dans lesquelles on a testé la quantité d'eau absorbée pour une même masse (100g) d'excipients de nature différente :

| NATURE DE L'EXCIPIENT | QUANTITE D'EAU ABSORBEE POUR 100G D'EXCIPIENT |
|---|---|
| . Lactose | ≈ 15g |
| . Amidon | ≈ 60g |
| . Cellulose microcristalline | ≈ 120g |
| . Ether de cellulose hydroxypropylé faiblement substitué (LHPC) | ≈ 350g |

La masse sèche de polymère cellulosique hydroxypropylé est mouillée avec un liquide d'humidification pouvant être la solution active ou un autre liquide aqueux ou non aqueux dans le cas d'une poudre active, jusqu'à l'obtention d'une pâte homogène et malléable pouvant subir les étapes suivantes du procédé, c'est-à-dire une extrusion et une sphéronisation.

Le liquide d'humidification sert de véhicule pour transporter et déposer les substances actives jusqu'au coeur de la substance absorbante et adsorbante.

Le procédé de fabrication consiste ensuite à extruder la masse humide à travers une filière à orifices calibrés, puis à rendre sphérique (sphéroniser) le produit extrudé.

Pendant le processus d'extrusion, la masse humidifiée est tassée et transformée par étirement en filaments compacts de section généralement cylindrique et définie appelés « extrudats ».

Pour obtenir des sphéroïdes, on place les « extrudats » dans un appareil cylindrique appelé « sphéroniseur » contenant en sa partie inférieure un disque cannelé tournant à une vitesse variable et contrôlée. Sous l'effet de la force centrifuge exercée par la rotation du disque tournant, les « extrudats » se fragmentent régulièrement, puis se transforment en sphères par un effet de roulage-liage.

Il a été constaté que la possibilité de transformer les extrudats en sphéroïdes de sphéricité homogène, de granulométrie régulière préalablement définie, dépend autant des caractéristiques plastiques des extrudats, donc des caractéristiques plastiques de la masse humidifiée, que des caractéristiques liées à l'opération de sphéronisation proprement dite, c'est-à-dire de la vitesse de rotation du disque tournant et de la durée de sa rotation.

Selon une caractéristique de l'invention, pour obtenir une plasticité adaptée, compatible avec les techniques d'extrusion puis de sphéronisation, la masse de liquide d'humidification est préférentiellement comprise entre 3 et 5 fois la masse de polymère cellulosique hydroxypropylé utilisée.

Une caractéristique particulière de l'invention citée à titre d'exemple consiste à préparer des sphéroïdes de granulométrie comprise le plus fréquemment entre 350 microns et 1250 microns.

Dans ce cas, la filière de l'extrudeuse comporte des orifices de 1000 microns de diamètre d'ouverture et de 1000 microns de longueur (épaisseur de la filière). La vitesse d'extrusion est de 136 rpm pour un appareil extrudeur de type frontal bivis ou autre.

Le cycle de sphéronisation pour un appareil de sphéronisation de 25 cm de diamètre ou autre est alors par exemple d'une durée de 5 minutes pour une vitesse de rotation de 1040 rpm.

Les sphéroïdes sont ensuite séchés à une température voisine de 30-40°C.

Ils sont ensuite passés à travers un dispositif de calibrage, constitué par exemple d'un ensemble de tamis, afin d'obtenir des systèmes multiparticulaires sphériques de granulométrie maîtrisée.

Les sphéroïdes obtenus sont stables dans le temps, faciles à contrôler et reproductibles. Ils sont d'utilisation simple et pratique. Ils peuvent être stockés sans précautions, ni soins particuliers et sont facilement transportables car ils sont légers, peu encombrants et peu fragiles.

Les sphéroïdes selon la présente invention peuvent être présentés tels quels ou enrobés en vrac, en distributeur-doseur, en gélules, comprimés, sachets ou sous toute autre forme physique appropriée ou conditionnement.

De façon préférentielle, ils sont regroupés en capsules ou gélules, correspondant à une quantité de substances actives déterminée en fonction de la posologie habituelle de la composition et permettant une prise unitaire plus facile.

Avant leur conditionnement, par exemple sous forme de gélules, ces systèmes multiparticulaires peuvent être pelliculés au cours d'une étape supplémentaire du procédé selon l'invention.

Cette opération, réalisée principalement lorsque les sphéroïdes sont destinés à une prise orale, consiste à les recouvrir d'un film de revêtement résistant permettant de protéger les molécules de principe actif par exemple sensibles au pH acide de l'estomac ou dégradées par les enzymes de la lumière intestinale.

Elle permet également de réguler la libération dans l'organisme des constituants actifs. La libération peut ainsi être retardée afin de délivrer les principes actifs de manière sélective à certains niveaux du système digestif, par exemple dans les parties supérieures et médianes de l'intestin grêle.

En utilisant des revêtements de différentes natures pour les sphéroïdes d'une même gélule, il est également possible de délivrer successivement plusieurs doses de principes actifs au moyen d'une prise unique de composition.

Selon une caractéristique essentielle de l'invention, le mélange technologique sec comprend un éther de cellulose hydroxypropylé faiblement substitué au niveau des groupes du noyau β-0-glucopyranosile, de préférence caractérisé par un taux de substitution de l'ordre de 10 % par exemple voisin de 11% et un pourcentage de fraction soluble dans l'eau voisin de 5 % par exemple 4,29%.

Outre ses propriétés plastiques, absorbantes et adsorbantes des liquides, compatibles avec le procédé d'extrusion-sphéronisation selon l'invention, ce composé présente une propriété particulièrement avantageuse de gonflement dans l'eau, catalysant la dispersion des substances actives absorbées et/ou adsorbées.

En effet, ce gonflement facilite l'entrée du liquide dans les microcavités de la matière absorbante et adsorbante et l'extraction par lessivage des produits actifs qu'elle porte.

Ce composé permet ainsi une dispersion par délitement rapide dans une solution aqueuse des composants adsorbés et/ou absorbés.

Grâce à ce moyen essentiel, il est possible d'atteindre un autre but de l'invention à savoir réaliser des sphéroïdes à dispersion rapide dans un milieu liquide comme l'eau notamment, avec l'aide ou non de la température. Le fluide obtenu peut être utilisé par voie interne ou externe pour résoudre des problèmes notamment dans les domaines précédemment cités dans l'introduction.

Ainsi, par exemple après une absorption orale du produit, une libération rapide des substances a lieu dans l'estomac, le liquide gastrique réalisant le lessivage nécessaire.

L'ensemble des principes actifs est libéré très rapidement, de manière contrôlée et reproductible. Cette biodisponibilité exceptionnelle rend cette formulation originale et d'une grande efficacité en médecine préventive et curative.

Le procédé de fabrication de sphéroïdes à dispersion rapide selon l'invention comprend alors les étapes suivantes :
- fournir une solution ou une poudre active ;
- fournir un mélange technologique homogène sec de polymère cellulosique hydroxypropylé faiblement substitué à propriétés absorbantes et adsorbantes compatibles avec le procédé d'extrusion et de sphéronisation et permettant une dispersion par délitement rapide des composants apportés ;
- effectuer l'humidification du mélange sec par la solution active ou par un autre liquide aqueux ou non aqueux ;
- extruder ;
- former des sphéroïdes par sphéronisation du produit extrudé ;
- sécher ;
- calibrer les sphéroïdes secs pour former des systèmes multiparticulaires sphériques de granulométrie maîtrisée.

Là encore ce procédé peut comporter une étape supplémentaire consistant à pelliculer les systèmes multiparticulaires.

Afin d'améliorer encore ce résultat avantageux de libération rapide et de conférer de nouvelles propriétés aux sphéroïdes, une caractéristique de l'invention consiste à utiliser les propriétés physico-chimiques et pharmaco-techniques particulières d'excipients compatibles avec la mise en forme des sphéroïdes, ainsi que leur aptitude à la dispersion en milieu liquide, l'eau notamment, par désagrégation rapide une fois au contact d'un fluide, d'une solution complexe ou de toute composition liquide, pâteuse ou semi-pâteuse à teneur en eau élevée.

Selon un mode de réalisation préférentiel de l'invention, l'éther de cellulose hydroxypropylé est associé avec au moins un excipient à usage pharmaceutique conventionnellement utilisé pour sa solubilité aqueuse performante, par exemple de type lactose ou autres dérivés, de préférence dans des proportions comprises sensiblement entre 20 et 50% de la masse sèche totale.

Il peut également être associé avec un excipient désintégrant à propriétés délitantes « flash », tel que la carboxyméthylcellulose sodique réticulée, de préférence dans des proportions voisines de 5% de la masse sèche totale.

La composition peut comprendre en outre pour lui conférer des propriétés supplémentaires d'autres excipients couramment utilisés avec les techniques actuelles, comme par exemple des liants, des agents glissants, des composés lubrifiants, des tensioactifs ou autres.

Les exemples suivants permettent de mieux comprendre l'invention.

Des sphéroïdes à base de matières premières d'origine végétale (extraits liquides), de titre alcoolique et de résidu sec variables, ont été préparés par le procédé selon l'invention et avec l'excipient technologique décrit ci-dessus.

### Exemple 1 :

- extrait liquide de valériane 350 ml
   titre alcoolique : 27%, résidu sec : 9,1%
- éther de cellulose hydroxypropylé hydroxypropylé faiblement substitué (LHPC) 100g
   faiblement substitué

### Exemple 2 :

- extrait liquide de vigne rouge hydroxypropylé faiblement substitué (LHPC) 350 ml
   titre alcoolique : 11%, résidu sec : 9,5%
- éther de cellulose hydroxypropylé 100g
   faiblement substitué

### Exemple 3 :

- extrait liquide de pissenlit 300 ml
   titre alcoolique : 40%, résidu sec : 21,0%
- éther de cellulose hydroxypropylé 100g
   faiblement substitué

## Revendications

1. Sphéroïdes **caractérisés en ce qu'**ils sont formulés à partir d'au moins une substance active absorbée et/ou adsorbée sur un mélange technologique homogène sec de polymère cellulosique hydroxypropylé faiblement substitué et qu'ils sont obtenus par un procédé d'extrusion et de sphéronisation.

2. Sphéroïdes selon la revendication précédente **caractérisés en ce que** la substance active est d'origine végétale.

3. Sphéroïdes selon la revendication précédente **caractérisés en ce que** la substance active provient initialement d'un extrait alcoolique ou hydro-alcoolique d'une ou de plusieurs matières premières végétales.

4. Sphéroïdes selon l'une quelconque des revendications précédentes **caractérisés en ce que** le mélange technologique comprend un éther de cellulose hydroxypropylé faiblement substitué au niveau des groupes du noyau β-O-glucopyranosile.

5. Sphéroïdes selon la revendication précédente **caractérisés en ce que** l'éther de cellulose hydroxypropylé présente un taux de substitution (groupements hydroxypropyls) de l'ordre de 10 % notamment voisin de 11%.

6. Sphéroïdes selon la revendication 4 ou 5 **caractérisés en ce que** l'éther de cellulose hydroxypropylé présente un pourcentage de fraction soluble dans l'eau de l'ordre de 5 % notamment voisin de 4,29%.

7. Sphéroïdes selon l'une quelconque des revendications 4 à 6 **caractérisés en ce que** l'éther de cellulose hydroxypropylé est associé avec au moins un excipient à usage pharmaceutique conventionnellement utilisé pour sa solubilité aqueuse performante et notamment avec du lactose.

8. Sphéroïdes selon la revendication précédente **caractérisés en ce que** cet au moins un excipient représente sensiblement 20 à 50% de la masse sèche totale.

9. Sphéroïdes selon l'une quelconque des revendications 4 à 8 **caractérisés en ce que** l'éther de cellulose hydroxypropylé est associé à un excipient désintégrant à propriétés délitantes « flash » et notamment avec de la carboxyméthylcellulose sodique réticulée.

10. Sphéroïdes selon la revendication précédente **caractérisés en ce que** cet excipient désintégrant représente environ 5% de la masse sèche totale.

11. Sphéroïdes selon l'une quelconque des revendications précédentes **caractérisés en ce qu'**ils sont regroupés dans des capsules ou gélules.

12. Composition végétale, minérale, vitaminique ou à base de composants actifs organiques **caractérisée en ce qu'**elle se présente sous une forme galénique de sphéroïdes selon l'une quelconque des revendications précédentes.

13. Procédé de fabrication de sphéroïdes fortement titrés en principes actifs **caractérisé en ce qu'**il comprend les étapes suivantes :
. fournir une solution ou une poudre active très concentrée ;
. fournir un mélange technologique homogène sec de polymère cellulosique hydroxypropylé faiblement substitué à propriétés absorbantes et adsorbantes très élevées ;
. effectuer l'humidification du mélange sec par la solution active ou par un autre liquide aqueux ou non aqueux ;
. extruder ;
. former des sphéroïdes par sphéronisation du produit extrudé ;
. sécher ;
. calibrer les sphéroïdes secs pour former des systèmes multiparticulaires sphériques de granulométrie maîtrisée.

14. Procédé de fabrication de sphéroïdes à libération rapide des substances actives, **caractérisé en ce qu'**il comprend les étapes suivantes :
. fournir une solution ou une poudre active ;
. fournir un mélange technologique homogène sec de polymère cellulosique hydroxypropylé faiblement substitué à propriétés absorbantes et adsorbantes compatibles avec le procédé d'extrusion et de sphéronisation et permettant une dispersion par délitement rapide des composants apportés ;
. effectuer l'humidification du mélange sec par la solution active ou par un autre liquide aqueux ou non aqueux ;
. extruder ;
. former des sphéroïdes par sphéronisation du produit extrudé ;
. sécher ;
. calibrer les sphéroïdes secs pour former des systèmes multiparticulaires sphériques de granulométrie maîtrisée.

15. Procédé de fabrication de sphéroïdes selon la revendication 12 ou 13 **caractérisé en ce qu'**il comprend en outre l'étape suivante :
. pelliculer ces systèmes multiparticulaires.

16. Procédé de fabrication de sphéroïdes selon l'une quelconque des revendications 12 à 14 **caractérisé en ce que** la masse de liquide d'humidification est comprise entre 3 et 5 fois la masse de polymère cellulosique hydroxypropylé utilisée.

17. Procédé de fabrication de sphéroïdes selon l'une quelconque des revendications 12 à 16 **caractérisé en ce que** les sphéroïdes sont séchés à une température voisine de 30-40°C.

18. Procédé de fabrication de sphéroïdes selon l'une quelconque des revendications 12 à 16 **caractérisé en ce que** les sphéroïdes secs sont calibrés de façon à obtenir des sphéroïdes de granulométrie comprise entre 350 microns et 1250 microns.

## Claims

1. Spheroids **characterized in that** they are formulated from at least one active substance absorbed and/or adsorbed on a dry homogeneous technological mixture of slightly substituted cellulosic hydroxypropylated polymer and **in that** they are obtained using an extrusion and spheronizing method.

2. Spheroids according to the preceding claim **characterized in that** the active substance is of vegetable origin.

3. Spheroids according to the preceding claim **characterized in that** the active substance initially originates from an alcoholic or hydro-alcoholic extract of one or more primary vegetable materials.

4. Spheroids according to any one of the preceding claims **characterized in that** the technological mixture comprises a slightly substituted cellulosic hydroxypropylated polymer ether at the level of the core groups β-O-glucopyranosil.

5. Spheroids according to the preceding claim **characterized in that** the hydroxypropylated cellulosic ether has a substitution rate (hydroxypropyl groups) of the order of 10%, specifically about 11%.

6. Spheroids according to claim 4 or 5 **characterized in that** the hydroxypropylated cellulosic ether has a water soluble fractional percentage of the order of 5%, specifically, approximately 4.29%.

7. Spheroids according to any one of claims 4 through 6 **characterized in that** the hydroxypropylated cellulosic ether is associated with at least one pharmaceutical excipient that is conventionally used for its high performance aqueous solubility, especially with lactose.

8. Spheroids according to the preceding claim **characterized in that** this at least one excipient generally represents from 20 to 50% of the total dry mass.

9. Spheroids according to any one of claims 4 through 8 **characterized in that** the hydroxypropylated cellulosic ether is associated with a disintegrating excipient possessing Φ flashΘ delitescent properties, particularly with reticulated sodic carboxymethylcellulose.

10. Spheroids according to the preceding claim **characterized in that** the disintegrating excipient represents about 5 % of the total dry mass.

11. Spheroids according to any one of the preceding claims **characterized in that** they are grouped in capsules or gelatin capsules.

12. A vegetable, mineral, vitamin, or active organic component-based compound **characterized in that** it takes the form of galenic spheroids according to any one of the preceding claims.

13. A method of fabricating spheroids heavily titrated with active principles, **characterized in that** it comprises the following steps:
. providing a highly concentrated active solution or powder;
. providing a dry homogeneous technological mixture of slightly substituted hydroxypropylated cellulosic polymer with very elevated absorbent and adsorbent properties;
. wetting the dry mixture with the active solution or with another aqueous or non-aqueous liquid;
. extrusion;
. formation of spheroids by spheronizing the extruded product;
. drying;
. calibrating the dried compound in order to form multiparticular spherical systems of controlled granular size.

14. A method of fabricating rapid dispersion spheroids **characterized in that** it comprises the following steps:
. providing an active solution or powder;
. providing a dry homogeneous technological mixture of slightly substituted hydroxypropylated cellulosic polymer with absorbent and adsorbent properties compatible with the extrusion and spheronization procedure and allowing rapid delitescent dispersion of the added components;
. wetting the dry mixture with the active solution or with another aqueous or non-aqueous liquid;
. extrusion;
. formation of spheroids by spheronizing the extruded product; drying
. calibrating the dried compound in order to form multiparticular spherical systems of controlled granular size.

15. A method of fabricating spheroids according to claim 12 or 13 **characterized in that** it further comprises the following step:
. coating the multiparticular systems with film.

16. A method of fabricating spheroids according to any one of claims 12 through 14 **characterized in that** the mass of the wetting liquid ranges from 3 to 5 times the mass of hydroxypropylated cellulosic polymer used.

17. A method of fabricating spheroids according to any one of claims 12 through 15 **characterized in that** the extrusion die comprises orifices with a 1000 micron diameter opening and 1000 microns long and **in that** the speed of extrusion is 100 rpm.

18. A method of fabricating spheroids according to any one of claims 12 through 16 **characterized in that** the spheronization cycle effected with a 25 cm diameter spheronization apparatus is 5 minutes long at a rotation speed of 1040 rpm.

19. A method of fabricating spheroids according to any one of claims 12 through 17 **characterized in that** the spheroids are dried at a temperature of approximately 30-40°C.

20. A method of fabricating spheroids according to any one of claims 12 through 18 **characterized in that** the dried compound is calibrated to yield spheroids ranging in granular size from 350 microns to 1250 microns, for a yield of at least 95% of the mass of spheroids produced.

## Patentansprüche

1. Sphäroide, **dadurch gekennzeichnet, dass** sie ausgehend von mindestens einer Aktivsubstanz hergestellt werden, die in einem technologischen homogenen Trockengemisch aus schwach substituierten hydroxypropylierten Zellulosepolymeren absorbiert und / oder adsorbiert wird, und dass sie durch ein Verfahren der Extrusion und der Sphäronisation erhalten werden.

2. Sphäroide nach dem vorhergehenden Anspruch, **dadurch gekennzeichnet, dass** die Aktivsubstanz pflanzlichen Ursprungs ist.

3. Sphäroide nach dem vorhergehenden Anspruch, **dadurch gekennzeichnet, dass** die Aktivsubstanz ursprünglich aus einem Alkohol- oder Hydroalkoholextrakt aus einem oder mehreren pflanzlichen Grundstoffen stammt.

4. Sphäroide nach einem der vorhergehenden Ansprüche, **dadurch gekennzeichnet, dass** das technologische Gemisch ein Äther der hydroxypropylierten Zellulose umfasst, das an den Gruppen des β-O-Glucopyranosil-Kerns schwach substituiert ist.

5. Sphäroide nach dem vorhergehenden Anspruch, **dadurch gekennzeichnet, dass** das Äther der hydroxypropylierten Zellulose einen Prozentsatz der Substituierung (Hydroxypropylgruppen) im Bereich von 10 % und insbesondere nahe 11 % aufweist.

6. Sphäroide nach einem der Ansprüche 4 oder 5, **dadurch gekennzeichnet dass** das Äther der hydroxypropylierten Zellulose einen Prozentsatz der in Wasser löslichen Fraktion im Bereich von 5 % und insbesondere nahe 4,29 % aufweist.

7. Sphäroide nach einem der Ansprüche 4 bis 6, **dadurch gekennzeichnet, dass** das Äther der hydroxypropylierten Zellulose mit mindestens einem pharmazeutischen Hilfsstoff für die pharmazeutische Anwendung assoziiert ist, der üblicherweise aufgrund seiner starken Wasserlöslichkeit verwendet wird, und insbesondere mit Laktose.

8. Sphäroide nach dem vorhergehenden Anspruch, **dadurch gekennzeichnet, dass** mindestens ein pharmazeutischer Hilfsstoff genau 20 bis 50 % der gesamten Trockenmasse ausmacht.

9. Sphäroide nach einem der Ansprüche 4 bis 8, **dadurch gekennzeichnet dass** das Äther der hydroxypropylierten Zellulose an einen pharmazeutischen Hilfsstoff assoziiert ist, der Eigenschaften der Flash-Verdampfung aufweist, und insbesondere mit retikulierter Natrium-Carboxymethylzellulose.

10. Sphäroide nach dem vorhergehenden Anspruch, **dadurch gekennzeichnet, dass** dieser zersetzende pharmazeutische Hilfsstoff etwa 5 % der gesamten Trockenmasse ausmacht.

11. Sphäroide nach einem der vorhergehenden Ansprüche, **dadurch gekennzeichnet, dass** sie in Kapseln oder Gelkapseln zusammengefasst sind.

12. Zusammensetzung aus Pflanzen, Mineralien, Vitaminen oder auf Basis von organischen Aktivsubstanzen, **dadurch gekennzeichnet, dass** sich diese in galenischer Form aus Sphäroiden nach einem beliebigen der vorhergehenden Ansprüche darstellt.

13. Herstellungsverfahren für Sphäroide, welche an aktiven Prinzipien stark titriert sind, **dadurch gekennzeichnet, dass** dieses die folgenden Schritte umfasst:
- Bereitstellen einer sehr konzentrierten Lösung oder eines Aktivpuders;
- Bereitstellen einer technologischen homogenen Trockenmischung aus hydroxypropylierten Zellulosepolymeren, die schwach substituiert sind und die sehr hohe Eigenschaften der Absorption und Adsorption aufweisen;
- Ausführen der Befeuchtung der Trockenmischung durch die Aktivlösung oder durch eine andere wässrige oder nicht wässrige Flüssigkeit;
- Extrudieren;
- Herausbilden der Sphäroide durch Sphäronisation des extrudierten Produktes;
- Trocknen;
- Kalibrieren der getrockneten zusammensetzung, um sphärische Multipartikelsysteme einer bekannten Korngröße hervorzubringen.

14. Herstellungsverfahren für Sphäroide mit schneller Dispersion, **dadurch gekennzeichnet, dass** dieses die folgenden Schritte umfasst:
- Bereitstellen einer Lösung oder eines Aktivpuders;
- Bereitstellen einer technologischen homogenen Trockenmischung aus hydroxypropylierten Zellulosepolymeren, die schwach substituiert sind und die Eigenschaften der Absorption und Adsorption aufweisen, die mit dem Verfahren der Extrusion und der Sphäronisation kompatibel sind, und die eine Dispersion mittels schneller Aufspaltung der eingebrachten Bestandteile gewährleistet;
- Durchführen der Befeuchtung der Trockenmischung durch die Aktivlösung oder durch eine andere wässrige oder nicht wässrige Flüssigkeit;
- Extrudieren;
- Herausbilden der Sphäroide durch Sphäronisation des extrudierten Produktes;
- Trocknen;
- Kalibrieren der getrockneten Zusammensetzung, um sphärische Multipartikelsysteme einer bekannten Korngröße hervorzubringen.

15. Herstellungsverfahren für Sphäroide nach Anspruch 12 oder 13, **dadurch gekennzeichnet, dass** dieses außerdem den folgenden Schritt umfasst:
- Beschichten dieser Multipartikelsysteme.

16. Herstellungsverfahren für Sphäroide nach einem der Ansprüche 12 bis 14, **dadurch gekennzeichnet, dass** die flüssige Befeuchtungsmasse eine Menge zwischen drei und fünf Mal der verwendeten Masse der hydroxypropylierten Zellulosepolymere beträgt.

17. Herstellungsverfahren für Sphäroide nach einem der Ansprüche 12 bis 15, **dadurch gekennzeichnet, dass** das Extrusionswerkzeug Öffnungen mit einem Öffnungsdurchmesser von 1000 Mikronen und einer Länge von 1000 Mikronen umfasst und dass die Extrusionsgeschwindigkeit 100 rpm beträgt.

18. Herstellungsverfahren für Sphäroide nach einem der Ansprüche 12 bis 16, **dadurch gekennzeichnet, dass** der Zyklus der Sphäronisation mit einer Sphäronisationsvorrichtung mit einem Durchmesser von 25 cm und während einer Dauer von 5 Minuten bei einer Rotationsgeschwindigkeit von 1040 rpm durchgeführt wird.

19. Herstellungsverfahren für Sphäroide nach einem der Ansprüche 12 bis 17, **dadurch gekennzeichnet, dass** die Sphäroide bei einer Temperatur um 30 bis 40 °C getrocknet werden.

20. Herstellungsverfahren für Sphäroide nach einem der Ansprüche 12 bis 18, **dadurch gekennzeichnet, dass** die getrocknete Zusammensetzung auf eine Weise kalibriert wird, so dass man Sphäroide mit einer Korngröße zwischen 350 Mikronen und 1250 Mikronen für einen Ertrag von mindestens 95 % der Menge der hergestellten Sphäroiden erhält.
